# EUROPEAN PATENT SPECIFICATION

(11) **EP 0 859 009 B1**
(45) Date of publication and mention of the grant of the patent: **30.11.2005**
(21) Application number: 98108130.0
(22) Date of filing: 07.06.1991
(51) Int. Cl.: C07K 14/475, C12N 15/19, C12P 21/02

(54) **Recombinant human hepatocyte growth factor and method for production thereof**
Rekombinanter menschlicher Hepatozytwachstumsfaktor und Verfahren zu seiner Herstellung
Facteur recombinant humain de croissance d'hépatocyte et son procédé de préparation

(30) Priority: 11.06.1990 JP 15247490
(43) Date of publication of application: 19.08.1998
(62) Divisional of application: 91109369.8
(73) Proprietor: Nakamura, Toshikazu, Fukuoka-shi, Fukuoka 812 (JP)
(72) Inventor: Nakamura, Toshikazu, Fukuoka-shi, Fukuoka 812 (JP); Hagiya, Michio, c/o Toyo Boseki K.K., Ohtsu-shi, Shiga 520-02 (JP); Seki, Tatsuya, c/o Toyo Boseki K.K., Ohtsu-shi, Shiga 520-02 (JP); Shimonishi, Manabu, c/o Toyo Boseki K.K., Ohtsu-shi, Shiga 520-02 (JP); Shimizu, Shin, c/o Toyo Boseki K.K., Ohtsu-shi, Shiga 520-02 (JP); Ihara, Izumi, c/o Toyo Boseki K.K., Ohtsu-shi, Shiga 520-02 (JP); Sakaguchi, Mariko, c/o Toyo Boseki K.K., Ohtsu-shi, Shiga 520-02 (JP); Asami, Osamu, c/o Toyo Boseki K.K., Ohtsu-shi, Shiga 520-02 (JP)
(74) Representative: von Kreisler, Alek

(56) References cited:
- EP-A- 0 117 058
- EP-A- 0 293 785
- EP-A- 0 412 557
- NAKAMURA T ET AL: "Molecular cloning and expression of human hepatocyte growth factor" NATURE., vol. 342, no. 6248, 1989, LONDON GB, pages 440-443, XP002069438
- DATABASE WPI Section Ch, Week 9142 Derwent Publications Ltd., London, GB; Class B04, AN 91-306734 XP002069439 & JP 03 204 899 A (OTSUKA PHARM CO LTD)

## Description

### FIED OF THE INVENTION

The present invention relates to recombinant expression vectors capable of expressing a base sequence which codes for a hepatocyte growth factor (hereinafter HGF), transformants thereof and a method for producing HGF.

HGF is expected to serve well for hepatocyte cultivation reagents, liver regeneration promoters, basic research on liver function, research on the action of various hormones and drugs on hepatocytes, research on the carcinogenesis mechanism of hepatoma, clinical diagnostic reagents using an antibody against said polypeptide and therapeutic drugs for liver diseases.

### BACKGROUND OF THE INVENTION

Traditionally, epithelial cell growth factor (EGF), fibroblast growth factor (FGF), nerve cell growth factor(NGF), platelet-derived growth factor (PDGF), endothelial cell growth factor (ECGF) and other polypeptides have been known to possess cell growth activity. In addition to these cell growth factors, a polypeptide which shows hepatocyte growth activity in vitro was partially purified from serum of rats with regenerated liver by Nakamura et al. in 1984, and named hepatocyte growth factor (hereinafter abbreviated as HGF).

Until the discovery of HGF, it had been impossible to cultivate hepatocytes in vitro; they showed no growth even in the presence of mammalian serum which allows vigorous growth of various lines of established cells, and they usually fell down off from the wall of cultivation vessel in about 1 week. In the presence of HGF, hepatocytes showed very good growth, and their cultivation became possible [Biochem. Biophys. Res. Commun., *122*, 1450 (1984)]. Other workers confirmed that this HGF activity was present also in blood after partial hepatectomy and in blood of fulminant hepatitis patients.

With this background, the present inventors have made a series of investigations of HGF separated and purified from rat platelets, and found that this rat platelet-derived HGF comprises two kinds of subunits and succeeded in identifying 27 amino acid residues of a partial amino acid sequence of HGF (Japanese Patent Application No. 311866/1988).

Morever, EP-A-0 412 557 and EP-A-0 462 277 (both documents being relevant under Art. 54 (3) (4) EPC) disclose the use of animal cells for the expression of the placenta derived hHFG gene, and a cDNA comprising a nucleotide sequence coding for the peptide of Fig. 3, respectively.

The native HGF is a polypeptide which is secreted in only trace amounts from liver tissues and platelets, and difficulty in obtaining material tissues renderes stable supply of HGF almost impossible. Specifically, activity of human HGF has been heretofore confirmed only in sera from patients with fulminant hepatitis. Thus, in order to use this HGF for hepatocyte cultivation, researches of hepatocytes and as agents for hepatic diseases, mass-production of HGF or a polypeptide possessing similar activity by gene recombination technology is desired.

### SUMMARY OF THE INVENTION

With the aim of solving the problems described above, the present inventors have made intensive investigations, and found that a cDNA containing the base sequence which codes for rat HGF polypeptide can be obtained from a cDNA library prepared from rat liver mRNA using an oligonucleotide synthesized on the basis of the amino acid sequence of rat platelet-derived HGF as the probe. Further, it was found that cDNA containing a base sequence coding for human HGF polypeptide could be obtained from a cDNA library prepared from human liver mRNA with the use of said rat-originated cDNA (Nature, *342*, 440, 1989) as the probe.

The present inventors have further conducted Northern hybridization with mRNA derived from various human tissues other than liver using a part or all of said human liver-derived cDNA as the probe and found that HGF-like transcript can be found in placenta and leukocyte mRNAs. The present inventors have isolated cDNA containing a base sequence coding for human HGF from a cDNA library prepared from leukocyte-derived mRNA to clarify the base sequence. Furthermore, the inventors have obtained a transformant by preparing a recombinant expression vector containing said cDNA and transforming with said recombinant expression vector, and found that human leukocyte-derived HGF gene is expressed by cultivating said transformant, which resulted in completion of the present invention.

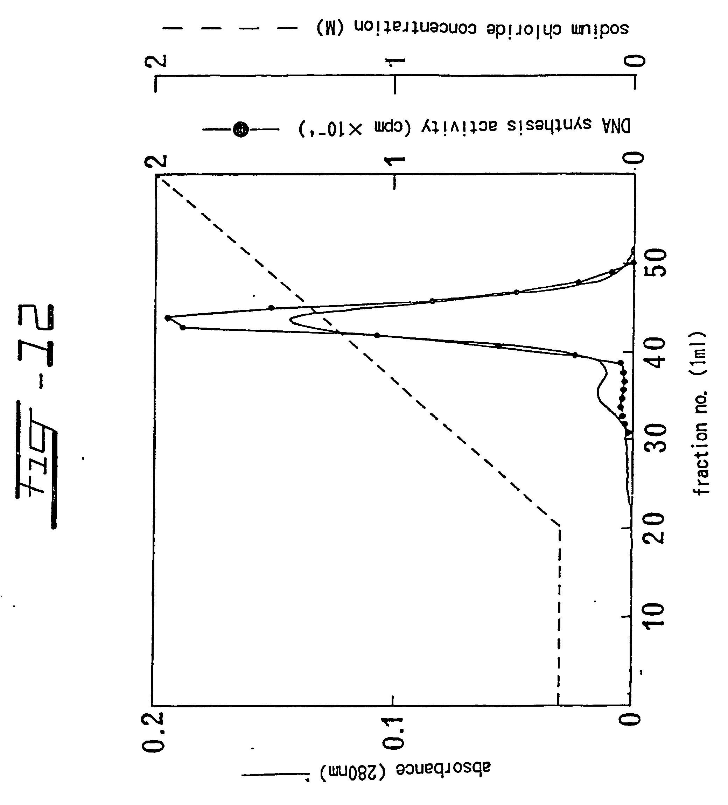

That is, this invention relates to
(1) a method of producing a hepatocyte growth factor (HGF) which comprises:
   1) transforming mammalian cells with a recombinant expression vector which has a promoter, a gene coding for an amino acid sequence shown in Fig. 2 and the DHFR gene,
   2) culturing the transformed cells in the presence of successively elevated concentrations of methotrexate, and
   3) harvesting the HGF from conditioned media of the cells obtained in 2);
(2) a transformant obtainable by transforming mammalian cells with a recombinant expression vector which has a promoter, a gene coding for an amino acid sequence shown in Fig. 2 and the DHFR gene; and
(3) a recombinant expression vector which has a promoter, a gene coding for an amino acid sequence shown in Fig. 2 and the DHFR gene.

### BRIEF EXPLANATION OF THE DRAWING

Fig. 1 shows a restriction enzyme map of HLC3.
Fig. 2 shows a part of the base sequence of HLC3 and therefrom deduced amino acid sequence.
Fig. 3 shows a part of the base sequence of HLC2 and therefrom deduced amino acid sequence.
Fig. 4 shows a construction schematic of human leukocyte-derived HGF expression vector for COS cell.
Fig. 5 shows a construction schematic of human leukocyte-derived HGF expression vector for mouse C127 cell.
Fig. 6 shows a construction schematic of human leukocyte-derived HGF expression vector for Chinese hamster CHO cell.
Fig. 7 is a line map showing fraction numbers of S-Sepharose eluate and absorbance of the eluted component and their relation to DNA synthesis activity.
Fig. 8 is a line map showing fraction numbers of heparin eluate and absorbance of the eluted component and their relation to DNA synthesis activity.
Fig. 9 is a line map showing relations between acetonitrile concentrations passed through reversed-phase HPLC and absorbance of the eluted component.
Fig. 10 shows SDS-polyacrylamide electrophoresis pattern of the purified recombinant human HGF under reducing and nonreducing conditions.
Fig. 11 is a line map showing chromatography pattern of the S-Sepharose eluate.
Fig. 12 is a line map showing fraction numbers of the heparin eluate and absorbance of the eluted component and their relation to DNA synthesis activity.
Fig. 13 is a line map showing fraction numbers of the phenyl 5PW column chromatography eluate and absorbance of the eluted component and their relation to DNA synthesis activity.
Fig. 14 shows SDS-polyacrylamide electrophoresis pattern of the purified recombinant human HGF under reducing and nonreducing conditions.
Fig. 15 shows SDS-polyacrylamide electrophoresis pattern of the purified single-chain recombinant human HGF under reducing and nonreducing conditions.

### DETAILED DESCRIPTION OF THE INVENTION

The DNA recombinant expression vector coding for human hepatocyte growth factor and the transformant of the invention can be prepared, for example, in the following manner.

That is, (1) mRNA is isolated from human leukocytes, from which a cDNA library is prepared by a known method;
(2) Using a portion or all of human liver-derived HGF cDNA which has been already isolated, as the probe, the above-mentioned human leukocyte-derived cDNA library is subjected to screening and the desired cDNA is extracted from the isolated clone;
(3) cDNA fragment coding for the human HGF is cut out from this human leukocyte-derived HGF and inserted into an expression vector;
(4) The obtained recombinant expression vector is used to transform a host cell to yield a transformant; and
(5) This transformant is cultivated, and human leukocyte HGF can be harvested and produced from the resulting culture supernatant.

The respective processes are in detail described below.

### (1) Isolation of mRNA and Northern hybridization

mRNA of human leukocytes can be obtained by a conventional method. For example, in accordance with the J. M. Chirgvin et al method as described in Biochemistry, *18*, 5294 (1979), said mRNA can be obtained by subjecting RNA obtained from the guanidine thiocyanate lysate of human leukocytes to liquid chromatography of oligo (dT) cellulose column or oligo (dT) latex. Also, human leukocyte mRNAs are available in the market, and the products of Clontech Lab. can be used. Northern hybridization of mRNA thus obtained and cDNA coding for human liver-derived HGF can be conducted, for example, by the Maniatis et al method as described in Molecular cloning : A Laboratory Manual, Cold Spring Harbor Laboratory, New York, *202* (1982). As the probe, all or a portion of human liver-derived HGF cDNA can be used after labeling with ³²P.

### (2) Preparation of cDNA

cDNA libraries can be constructed by synthesizing cDNAs using a reverse transcriptase with the human leukocyte mRNA wherein HGF transcript is confirmed as a template, in accordance with, for example, the H. Okayama et al method (Mol. Cell. Biol., *2*, 161. 1982 and Mol. Cell. Biol., *3*, 280, 1983) or the U. Gubler et al method (Gene, *25*, 263, 1983), and inserting said cDNAs into plasmids or phage DNAs. Examples of the plasmid vectors into which-the cDNAs are inserted include *Escherichia coli*-derived pBR322, pUC18 and pUC19 (Toyobo) and *Bacillus subtilis*-derived pUB110 (Sigma). The above-mentioned examples of the vectors to be used are not limited and any vectors can be used as long as they are capable of replication and amplification in host cells. As the methods in which the cDNAs synthesized from mRNA as the template are inserted into plasmids or phage DNAs to produce the cDNA libraries, mention can be made of, for example, the T. Maniatis' method (Molecular Cloning, Cold Spring Harbor Laboratory, New York, 1982, p.239) or the T. V. Hyunh et al method (DNA Cloning : A Practical Approach, *1*, 49, 1985). Also, human leukocyte cDNA libraries are available in the market like mRNAs and can be purchased from Clontech Corp. and others.

### (3) Screening of cDNA library

The recombinant expression vectors of plasmids or phage DNAs obtained as a cDNA library is harbored in an appropriate host cell such as *Escherichia coli.* Examples of *Escherichia coli* as the host cell include *Escherichia coli* NM514, C600 (Stratagene), NM522 and JM101 (Pharmacia). Using the calcium chloride method, the calcium chloride-rubidium chloride method or another method in the case where plasmids are used as the cDNA vector and using the *in vitro* packaging method in the case where phage DNAs are used as the cDNA vector, the recombinant expression vectors can be harbored in a previously grown host cell (Molecular Cloning, Cold Spring Harbor Laboratory, New York, 1982, p. 249). From the thus-obtained transformant, cDNA clones can be fished using ³²P-labeled human liver-derived HGF cDNA as the probe by the colony-hybridization method (Gene, *10*, 63, 1980) or the plaque hybridization method (Science, *196*, 180, 1977). The cloning can be conducted also by the enzyme antibody method (DNA Cloning, A Laboratory Manual: A Practical Approach, *1*, 49, 1985) with an antibody against the objective polypeptide to give the cDNA clones.

The recombinant DNA such as plasmid or or phage DNA is isolated from said transformant in accordance with the conventional method (Molecular Cloning, Cold Spring Harbor Laboratory, New York, 1982), whereafter the cDNA nucleotide sequence is determined directly as it is or after digested with restriction enzyme. The base sequence is determined by the Maxam-Gilbert chemical method [Proc. Natl. Acad. Sci. USA, *74*, 560 (1977)] or the Sanger dideoxy method [Proc. Natl. Acad. Sci. USA, *74*, 5463 (1977)]. Furthermore, another cDNA is synthesized newly from the above-mentioned mRNA by the primer-extension method (Proc. Natl. Acad. Sci. USA, *76*, 731, 1979) using as the primer a part of the cDNA whose sequence is determined or a synthesized DNA which is a part of the cDNA, and the cloning of the recombinant DNA of plasmid or phage containing the cDNA which can be ligated with a first cDNA obtained already from the cDNA library can be performed in the same manner as mentioned above. These steps of primer-extension and cloning can be repeated multiple times, if necessary.

### (4) construction of human HGF recombinant expression vector

A recombinant expression vector can be prepared by cutting out cDNA using restriction enzyme from several kinds of plasmid, phage or other recombinant vector containing the cloned chromosome DNA which codes for the entire or partial amino acid sequence of human leukocyte HGF and re-ligating it to the downstream of a vector promoter suitable for human leukocyte-derived HGF expression using restriction enzyme and DNA ligase.

More specifically, for increased expression efficiency of the human leukocyte-derived HGF, the recombinant expression vector is constructed so that 1) a promoter, 2) a ribosome binding site, 3) an initiation codon, 4) a DNA containing the base sequence which codes for the human leukocyte-derived HGF, 5) a termination codon and 6) a terminator are present therein in this order in the downstream direction of transcription. As the DNA vectors to be used in the present invention, mention can be made of, for example, *Escherichia coli*-derived plasmid pBR322, pUC18 (Toyobo), *Bacillus subtilis* -derived plasmid pUB110 (Sigma), yeast-derived plasmid pRB15 (ATCC 37062), bacteriophage λ gt10, λ gt11, (Stratagene), Virus SV40 (BRL Corp.), BPV (ATCC VR-703), retrovirus gene-derived vectors and so on. Any DNA vector can be used for the present invention as long as it is replicable and amplifiable in the host. Particularly, for convenient expression of the human leukocyte-derived HGF, it is preferable to use a vector derived from the gene of a virus such as SV40. For example, the recombinant expression vector wherein the above-mentioned cloned DNA which codes for human leukocyte-derived HGF is ligated to the late region of SV40 vector can be expressed by incorporating it into a simian cell line known as COS cells [Cell, *23*, 175 (1981)]. As for promoters and terminators, there is no limitation as long as they suit to the host used to express the desired base sequence which codes for human leukocyte-derived HGF. For example, there can be mentioned virus-derived SV40 promoter and HSV1 TK promoter for animal cell host such as mouse fibroblast and Chinese-hamster ovary cell. As the terminator, there can be mentioned, for example, SV40 terminator and HSV1 TK terminator for animal cell host. These promoters and terminators are used in appropriate combination according to the host used. The DNA containing a base sequence which codes for the human leukocyte-derived HGF is not subject to limitation as long as said DNA codes for the polypeptide which possesses hepatocyte growth activity, and has the sequence as shown in Fig. 2 to be mentioned later can be exemplified. DNA containing the base sequence which codes for the human leukocyte-derived HGF may contain a translation initiation codon ATG and a translation termination codon TAA, TGA or TAG. Also, if necessary, more than one initiation codon or termination codon may be used in combination, or may be combined with another codon, and these combinations are subject to no limitation. Moreover, the vector contains the DHFR gene capable of serving as a selection marker for the host transformed with this recombinant expression vector at an appropriate position.

### (5) Host cell transformation and cultivation

The human HGF leukocyte recombinant expression vector thus constructed is introduced into the host by the competent cell method [J. Mol. Biol., *53*, 154 (1970)], the protoplast method [Proc. Natl. Acad. Sci. USA, *75*, 1929 (1978)], the calcium phosphate method [Science, *221*, 551 (1983)], the DEAE dextran method [Science, *215*, 166 (1983)], the electric pulse method [Proc. Natl. Acad. USA, *81*, 7161 (1984)], the *in vitro* packaging method [Proc. Natl. Acad. Sci. USA, *72*, 581 (1975)], the virus vector method [Cell, 37, 1053 (1984)] or the microinjection method [Exp. Cell. Res., *153*, 347 (1984)]. Preferable mammalian host cells are mouse fibroblast C127 [J. Viol., *26*, 291 (1978)] and Chinese hamster ovarian cell CHO [Proc. Natl. Acad. Sci. USA, *77*, 1929 (1978)].

The obtained transformant is cultivated in a culture medium suitable to the host to produce the desired recombinant human leukocyte HGF. The medium is supplemented with carbon sources, nitrogen sources, minerals, vitamins, serum, chemicals and other additives necessary for the growth of the transformant. Examples of the culture medium include MEM medium, DMEM medium, RPMI1640 medium (Nissui Pharmaceutical) and so on containing fetal bovine serum in a proportion of not more than 20%.

Transformant cultivation is carried out normally at a temperature of 20 to 45°C and a pH of 5 to 8, with aeration and/or stirring added as necessary. When the host is an adhesive animal cell, glass beads, collagen beads, acetylcellulose hollow fiber or another carrier is used.

Medium compositions or cultivation conditions other than these may be used as long as they allow the transformant to grow.

### (6) Purification of human HGF

The recombinant human leukocyte HGF thus produced in the transformant or in the culture supernatant thereof may be separated and purified by a combination of known techniques such as salting-out, solvent precipitation, dialysis, ultrafiltration, gel electrophoresis, gel filtration chromatography, ion exchange chromatography, reverse phase chromatography and affinity chromatography. Particularly preferred efficient methods are the combination of ammonium sulfate salting-out, S-Sepharose ion chromatography, heparin Sepharose affinity chromatography and phenyl Sepharose reverse phase chromatography and the combination of ammonium sulfate salting-out, S-Sepharose ion chromatography and anti-HGF antibody Sepharose affinity chromatography.

The recombinant human leukocyte-derived HGF thus obtained showed noticeable promoting activity on the growth of rat hepatocytes like rat liver-derived HGF, rat platelet-derived HGF and recombinant human liver-derived HGF.

### (7) Determination of HGF activity

HGF activity was determined in accordance with the method described in Proc. Natl. Acad. Sci. USA, *80*, 7229 (1983) as follows: Hepatocytes were separated and purified from Wistar rats by the collagenase reflux method. The obtained rat hepatocytes were suspended in William E medium (Flow Laboratory) supplemented with 5% bovine serum, 2 × 10⁻⁹ M insulin and 2 × 10⁻⁹ M dexamethasone and sown over 24-well multiplates at a density of 1.25 × 10⁵ cells/well. After cultivation in the presence of 5% CO₂, 30% O₂ and 65% N₂ at 37°C for 20 hours, the medium was exchanged with William E medium supplemented with 0.1 µg/ml aprotinin, with simultaneous addition of a given amount of the subject sample. Fifteen hours later, 15 µCi/ml ¹²⁵I deoxyuridine was added at 10 µl/well. For the control group, 5 µg/ml aphidicolin was added 15 minutes before addition of ¹²⁵I deoxyuridine. Cultivation was continued for 4 more hours for ¹²⁵I labeling. After washing with two portions of PBS, pH 7.4, the cells were fixed in a cold 10% aqueous solution of trichloroacetic acid (TCA). The cells were solubilized with a 0.5 ml/well 1 N aqueous solution of sodium hydroxide, and their radioactivity was determined using a gamma counter. Also, a portion of the radioactivity-determined sample was taken and subjected to protein content determination by the Lowry method [J. Biol. Chem., *193,* 265 (1951)]. The amount of ¹²⁵I uptake into hepatocytes upon addition of the subject sample was calculated as the count difference from the control, and the obtained value was converted to per mg rat hepatocyte protein to obtain the DNA synthesis activity (dpm/mg protein). The HGF activity of the subject sample corresponding to 50% of the DNA synthesis activity of hepatocytes obtained with 10 ng/ml epithelial cell growth factor in the same test was defined as 1 unit.

The present invention enables a large quantity supply of novel physiologically active peptide which offers the growth of hepatocytes *in vitro*. The recombinant human leukocyte-derived HGF is useful as clinical diagnostic reagents and therapeutic agents for hepatic diseases. Moreover, hepatocytes grown and maintained by the action of the recombinant human leukocyte-derived HGF of the invention are extremely useful as host cells for basic researches of hepatic functions, researches of actions of various hormones and medicaments on hepatocytes, researches of carcinogenesis of hepatoma or the growth of hepatocytes *in vitro*.

The present invention is hereinbelow described in detail by illustrating working examples, to which the invention is not limited.

### Example 1

### 1) Northern hybridization of human tissue mRNA and human liver-derived HGF cDNA

Human brain, placenta, leukocyte, lung and liver mRNAs (2 µg each, Clontech Lab.) were subjected to 0.66 M formaldehyde-containing agarose gel electrophoresis according to the Maniatis et al method [Molecular cloning : A Laboratory Manual, Cold Spring Harbor, New York, *202* (1982)], followed by immobilization on a nylon filter gene screen plus (Dupont). The nylon filters were immersed in a hybridization solution containing a probe prepared by isolation and purification of BamHI-KpnI 2.2 kb fragment of human liver-derived HGF cDNA by agarose gel electrophoresis and labeling with [α³²P]dCTP using multi prime DNA labeling system (Amersham Corp.), 5 × SSPE buffer (1 × SSPE: 180 mM NaCl 10 mM sodium phosphate, 1 mM EDTA, pH 6.8), 5 × Denhardt solution, 10% dextran sulfate, 40% formaldehide, 0.1% SDS and 0.1 mg/ml *Escherichia coli* DNA, and subjected to hybridization reaction at 42°C for 16 hours. After the reaction, the nylon filters were washed three times with 1 × SSC buffer containing 0.1% SDS at 60°C and air-dried. The nylon filters were adhered to sensitization screens, lightning plus (Dupont), and X-ray films, RX (Fuji Photo Film), and exposed at -80°C for 16 hours. Upon development, HGF-like transcripts were confirmed in placenta and leukocyte mRNAs as in liver mRNA.

### 2) Preparation of human leukocyte-derived cDNA library:

cDNA was synthesized in accordance with the Gubler et al method (Gene, *25*, 263, 1983), using cDNA synthesis system plus (Amersham Corp.), wherein human leukocyte mRNA (3 µg) was used as the template, and oligonucleotide having a base sequence of ^{5'}ACATTCTCTGAAATCTTCAT^{3'} (SEQ ID No:1) present in 3' nontranslation region of human liver-derived HGF cDNA was used as the primer. After the cDNA was extracted with phenol/chloroform (1: 1, v/v) and purified by ethanol precipitation, it was dissolved in 10 mM Tris-HCl buffer containing 0.5 M NaCl and 1 mM EDTA (abbreviated as STE buffer) and the concentration of the solution was adjusted to 0.7 µg/20 µl. The cDNA was ligated with cDNA cloning system λ gt10 (Amersham Corp.) at the EcoRI site λ gt10 in accordance with the Huynh et al method (DNA Cloning I, A Practical Approach, *1*, 49, 1982) in the following manner. EcoRI adaptor was ligated with the cDNA at the both termini with the use of T4DNA ligase. The reaction mixture was applied onto a gel filtration column for cDNA purification which had been equilibrated with STE buffer. Upon elution with STE buffer, 500 µl of the cDNA fractions were collected. After ethanol precipitation was repeated twice by a conventional method, the linker-attached cDNA was obtained by drying under reducing pressure. The cDNA was again dissolved in STE buffer at a concentration of 50 ng/µl, and 0.1 µg of the adaptor-attached cDNA was inserted into 1 µg of λ gt10 vector prepared in advance, with the use of T4DNA ligase. After the reaction mixture was treated with cold ethanol, it was lightly dried and the entire amount of the obtained recombinant DNA was dissolved in 5 µl of 10 mM Tris-HCl buffer containing 1 mM EDTA, pH 7.5 (abbreviated as TE buffer). This recombinant DNA was subjected to *in vitro* packaging reaction to give λ gt10 recombinant phage. The number of the recombinant phages obtained from 1 µg of cDNA as measured by titration with *Escherichia coli* for phage-plating was 5.0 × 10⁶. The thus-obtained cDNA library was preserved until use in SE buffer with a slight amount of chloroform added (100 mM NaCl, 10 mM MgSO₄ and 20 mM Tris-HCl buffer containing 0.01% gelatin, pH 7.5) at 4°C.

### 3) Isolation of human leukocyte-derived HGF cDNA and determination of the nucleotide sequence

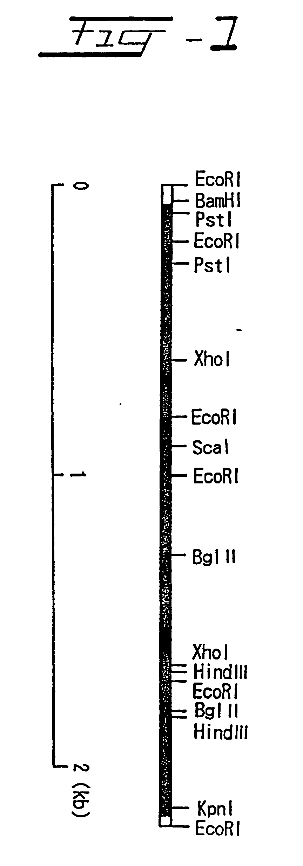

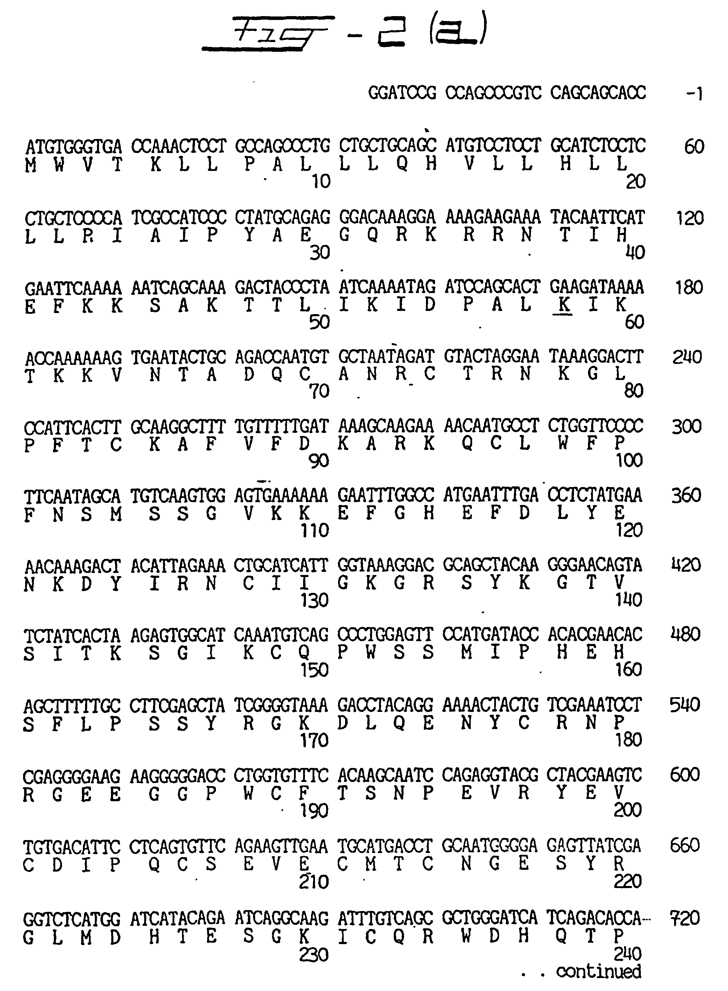

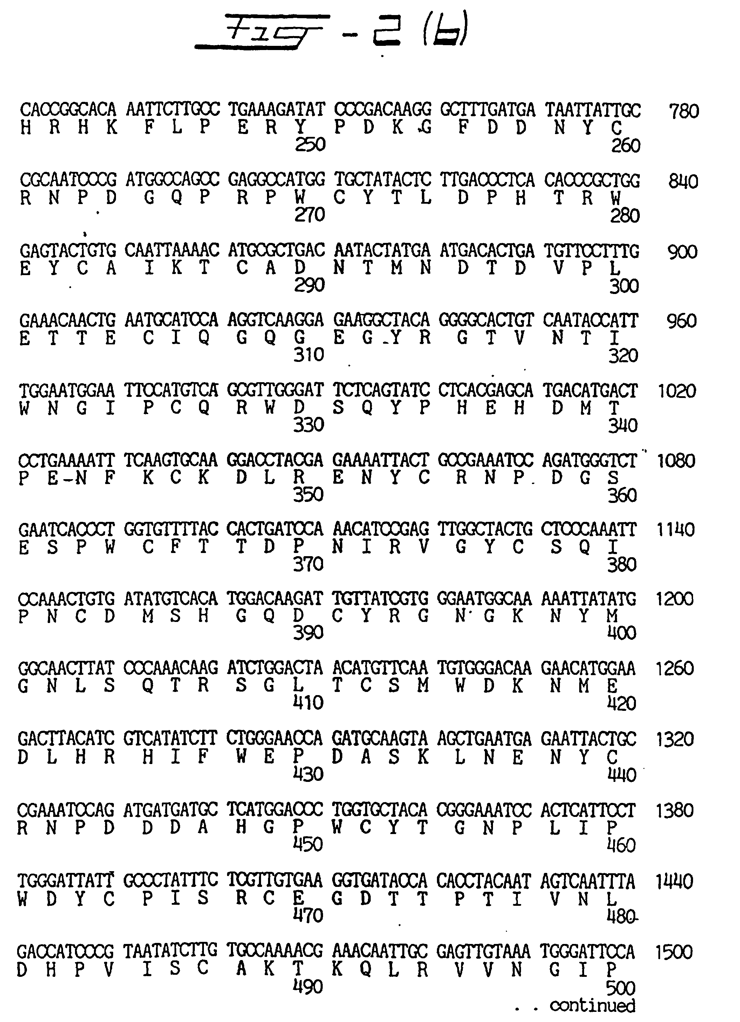

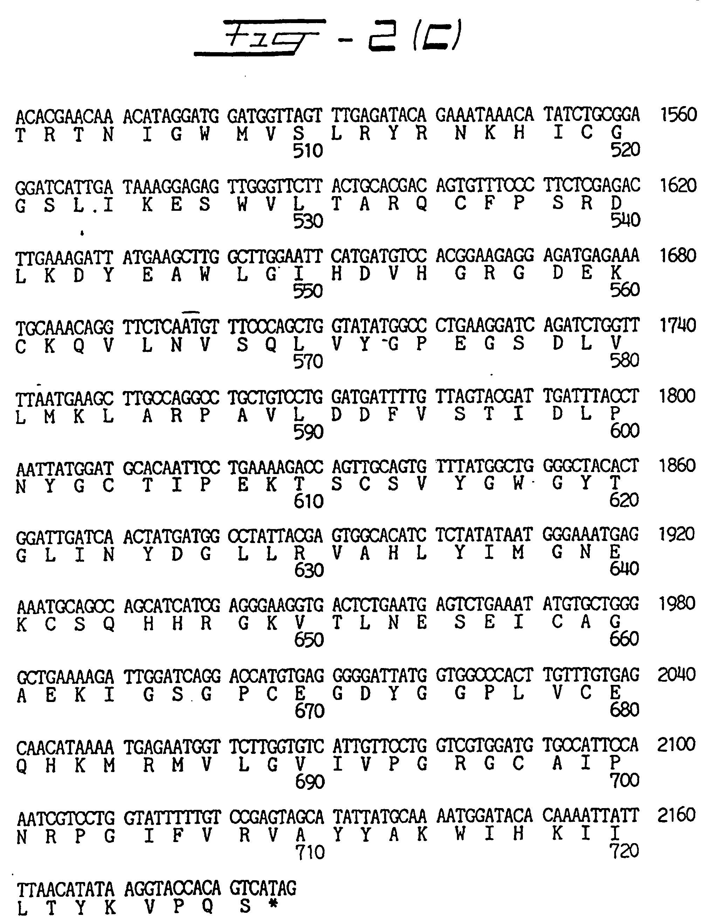

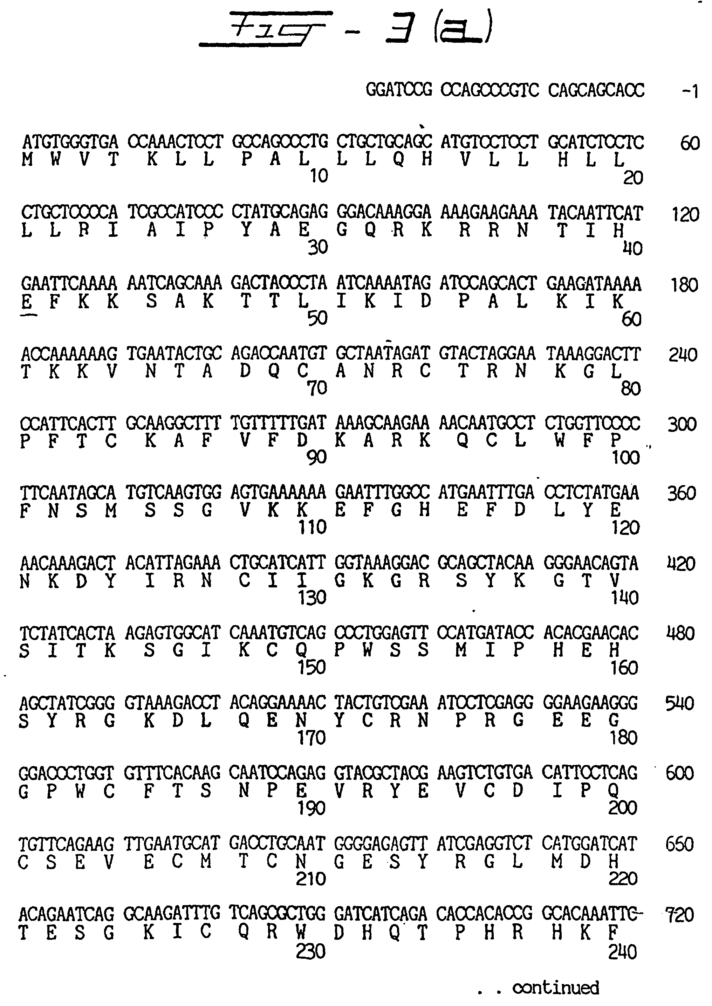

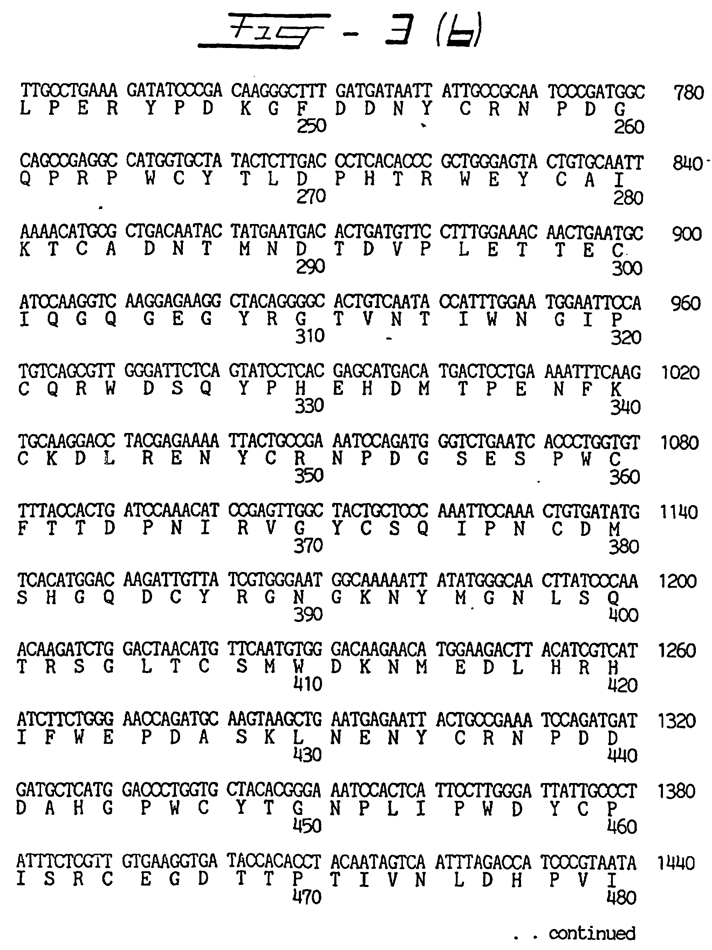

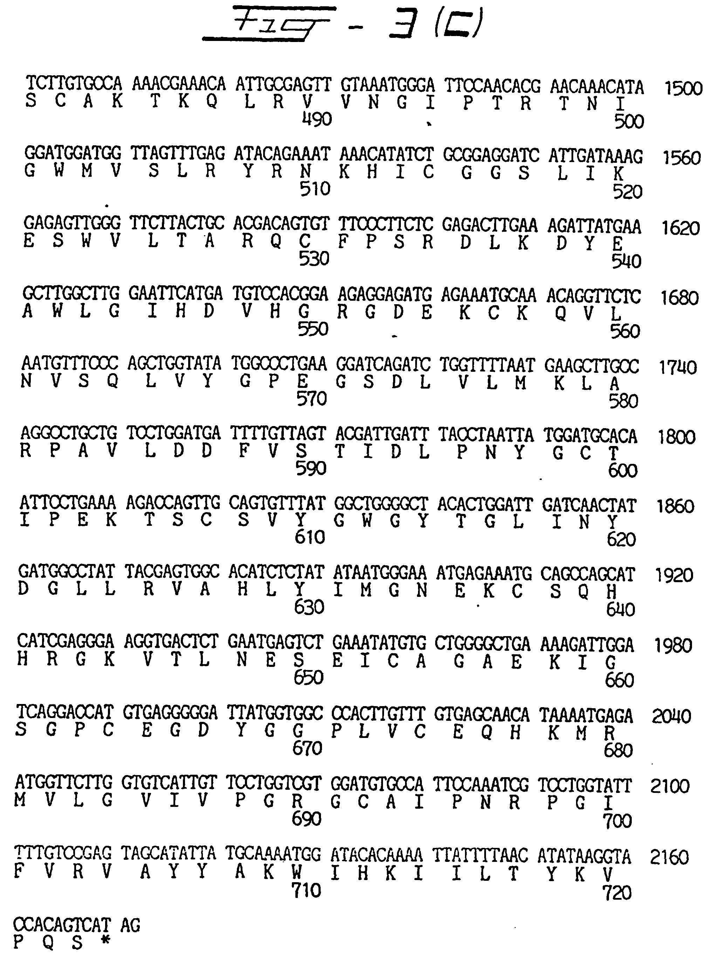

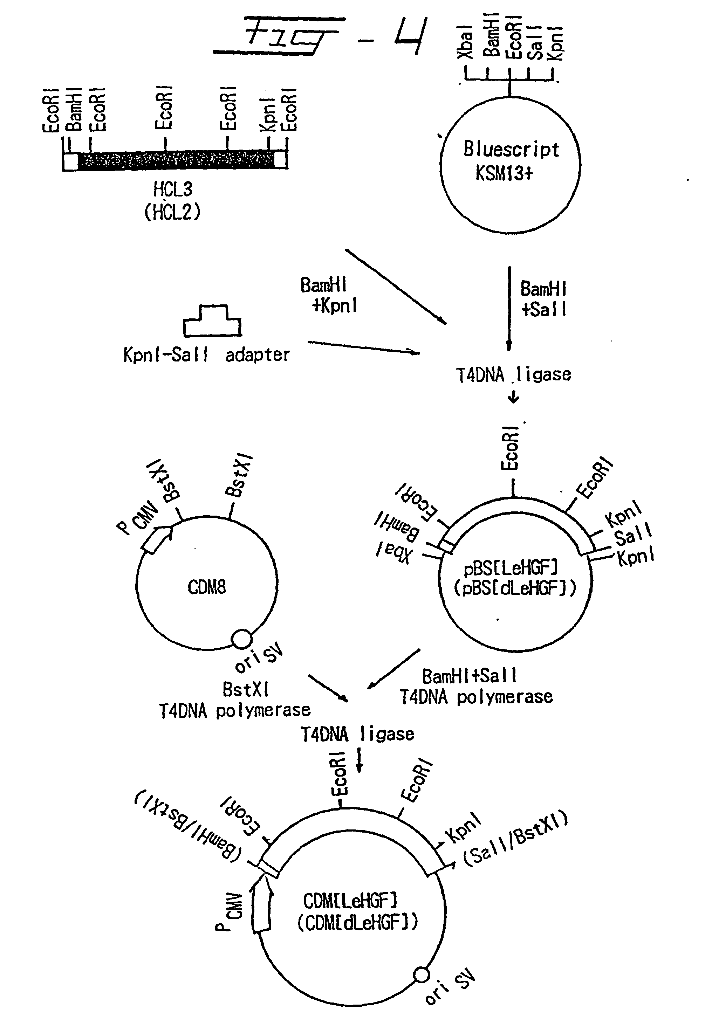

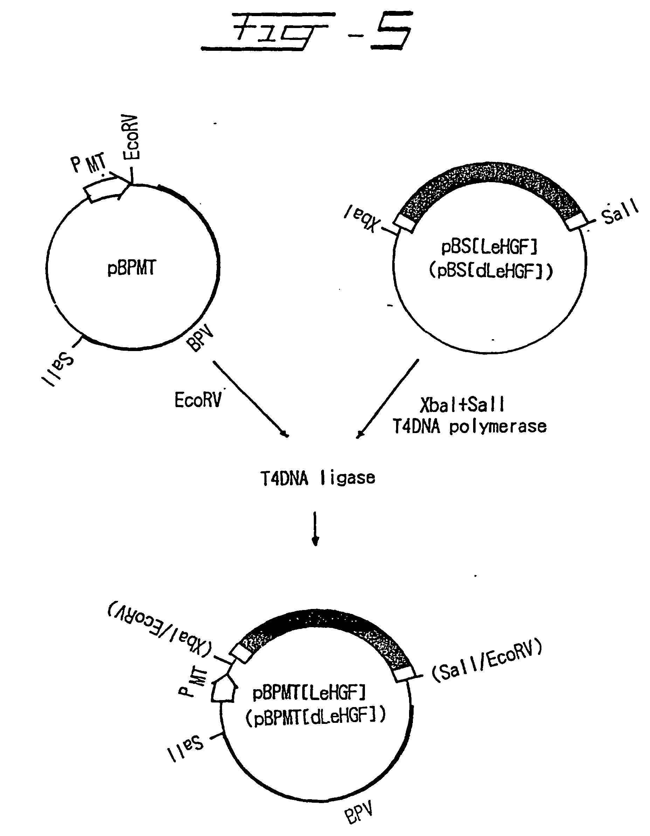

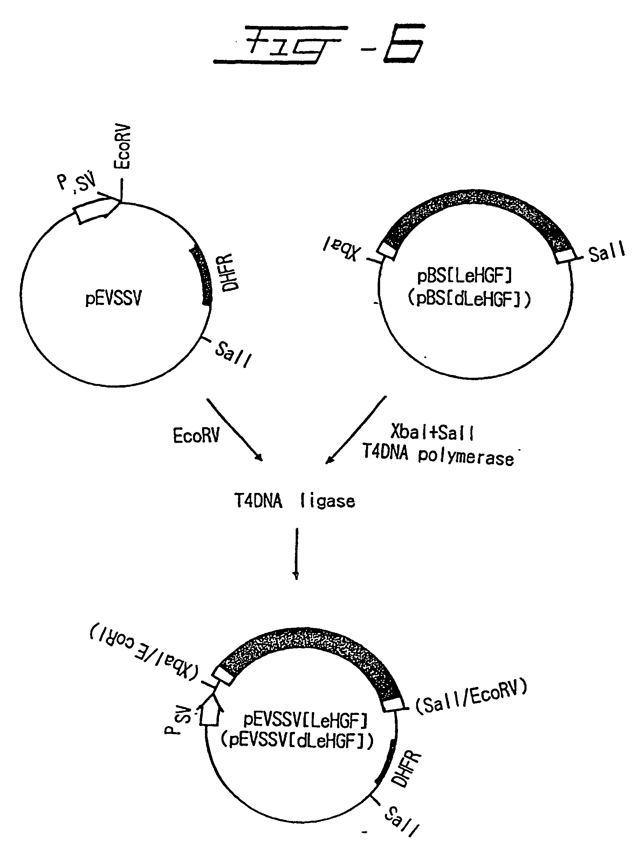

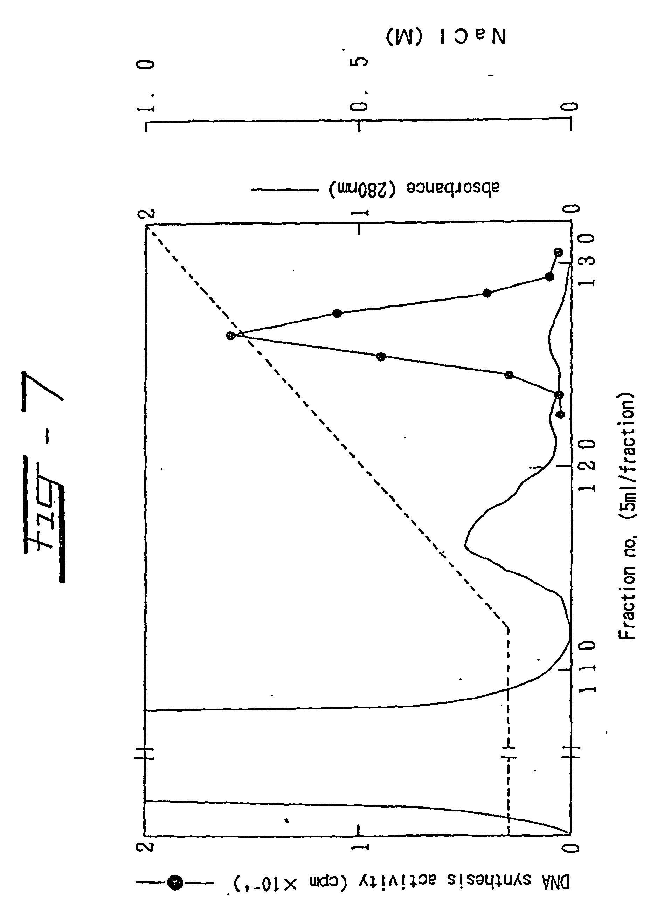

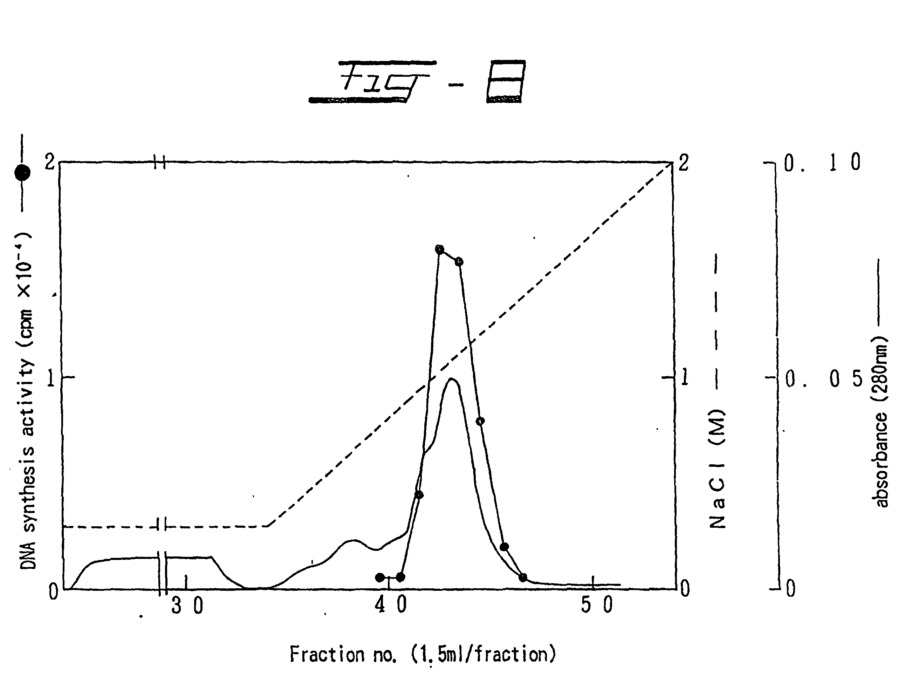

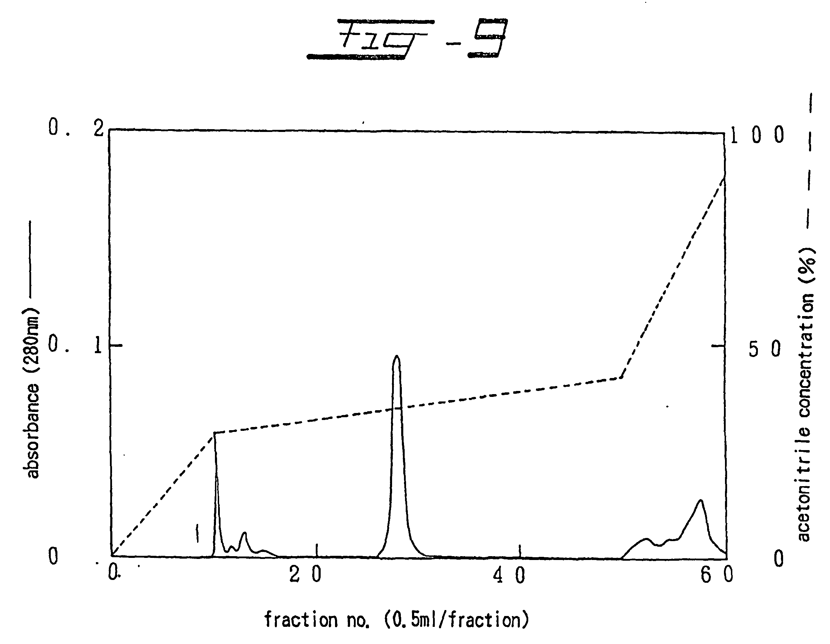

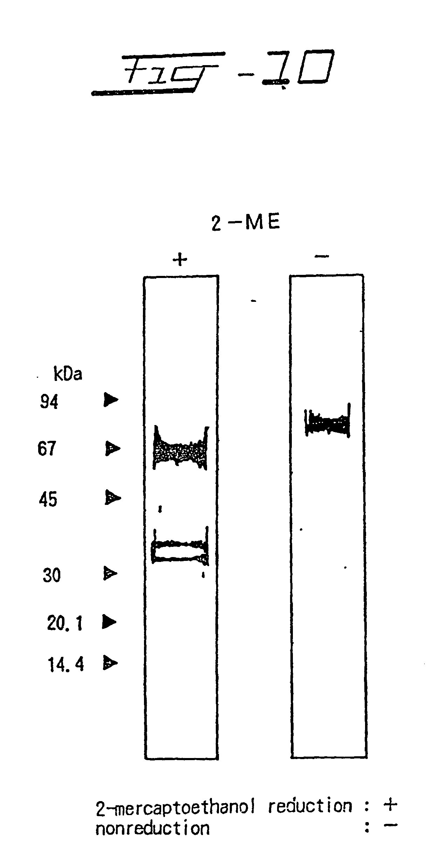

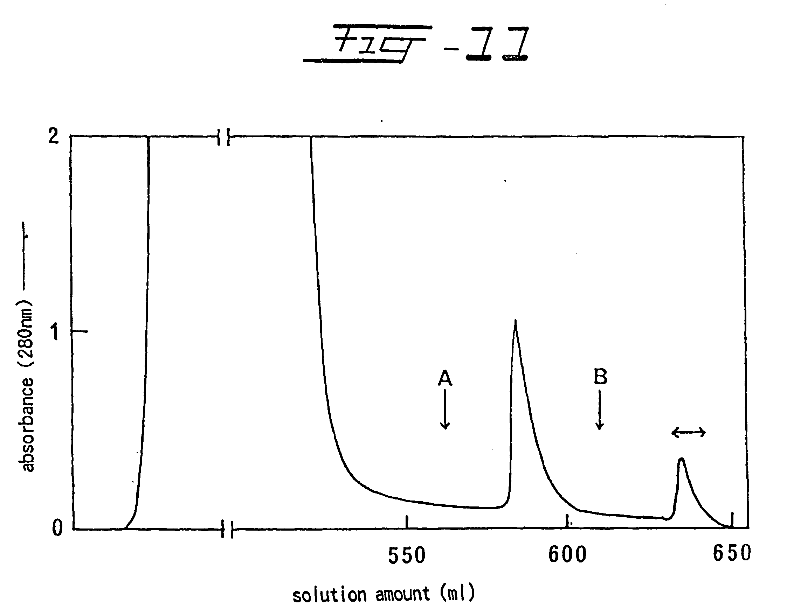

Cloning of human leukocyte-derived HGF gene from the above-mentioned cDNA library was conducted using 0.2 kb EcoRI fragment of HAC 69 as a probe which is a part of human liver-derived HGF cDNA labeled with [α³²P]dCTP by multi prime DNA labeling system (Amersham Corp.). Screening was conducted at a hybridization temperature and a washing temperature of 60°C and using 2 × SSC buffer containing 0.1% SDS to give positive clones HLC2 and HLC3. HLC2 and HLC3 cDNAs isolated and purified from each phage by a conventional method were subjected to restriction enzyme cleavage analysis and nucleotide sequence determination. In Fig. 1, shown is the restriction enzyme map of HLC3, and in Fig. 2, shown are a portion of the nucleotide sequence and the amino acid sequence deduced therefrom (SEQ ID No:2). The human leukocyte-derived HGF clone, HLC 3 shows similar characteristics as the human liver-derived HGF identified earlier (Nature, *342*, 440, 1989). Nevertheless, there are 38 different portions in the nucleotide sequence Th the coding region which causes 14 differences in the deduced amino acid sequence. HLC2 cDNA has almost the same nucleotide sequence as the HLC3 cDNA except the nucleotide sequence from the 483rd to the 497th which has been deleted (Fig. 3) (SEQ ID No:3).

### 4) Construction of human leukocyte-derived HGF expression vector for simian COS cell:

Construction schematics of human HGF expression vectors CDM[dLeHGF] and CDM[LeHGF] for simian COS cells are shown in Fig. 4. The HLC2 and HLC3 phage DNAs obtained in 3) above were digested with restriction enzymes BamHI and KpnI, and the 2.2 kb DNA fragment was isolated and purified. The oligonucleotides 5' CACAGTCATAGCTGTTAACCCGGG3' SEQ ID No:4 5' TCGACCCGGGTTAACAGCTATGACTGTGGTAC3' SEQ ID No:5 consisting of KpnI cleavage site of HLC2 and HLC3, a part of the sequence contained on its 3' region and HpaI, SmaI and SalI cleavage sites, were synthesized, which were then used as a KpnI-SalI adaptor. The BamHI-KpnI DNA fragments of HLC2 and HLC3 as described, the KpnI-SalI adaptor and Blue Script KSM13+ (Stratagene) which had been digested in advance with restriction enzymes BamHI and SalI were mixed, and ligated by T4DNA ligase to obtain two plasmids, pBS[dLeHGF] and pBS[LeHGF]. pBS[dLeHGF] and pBS[LeHGF] thus obtained were digested with restriction enzymes BamHI and SalI and given blunt ends with T4DNA polymerase, after which they were mixed with the expression vector CDM8 for COS cells (Nature, *329*, 840, 1987) which had been digested in advance with restriction enzyme BstX1 and given blunt ends with T4DNA polymerase, and ligated by T4DNA ligase to give human leukocyte-derived HGF expression vectors CDM[dLeHGF] and CDM[LeHGF] respectively.

### 5) Transformation of simian COS cell and expression of human leukocyte-derived HGF gene:

The obtained CDM[dLeHGF] and CDM[LeHGF] plasmids were subjected to ethanol precipitation, and thereafter dissolved in 10 mM PBS buffer and the concentration was adjusted to 2 µg/ml. The COS-1 cells (ATCC CRL-1650) which had been cultivated in DMEM medium (Nissui Pharmaceutical) containing 10% fetal bovine serum (Gibco) to saturated cell density, were washed twice with 10 mM PBS buffer and then subjected to trypsin treatment. After the cells were washed three times with said buffer, they were suspended in the buffer at the density of 2 × 10⁷ cell/ml. The plasmid solution (250 µl) and the cell suspension (250 µl) prepared previously were mixed and the mixture was left standing on ice for 10 minutes. High voltage pulse was given to the ice-cooled mixture of the plasmid and the cells at the applied voltage of 4 kV/cm during the pulse time of 20 m second by high-voltage-pulse gene-introducing apparatus ZA-1200 (PDS Corp.). The obtained cells were diluted in the above medium and cultured at 37 °C in the presence of 5% CO₂ for 3 days. HGF activity in the culture supernatant after 3 days' cultivation was determined and found to be 20 unit/ml and 5 unit/ml, respectively. By contrast, the expression vector CDM8 in which the HGF cDNA was not inserted was introduced into COS-1 cells and cultured in the same manner, but no HGF activity was detected in the culture supernatant.

### Example 2

### 1) Construction of human leukocyte-derived HGF expression vector for mouse C127 cells:

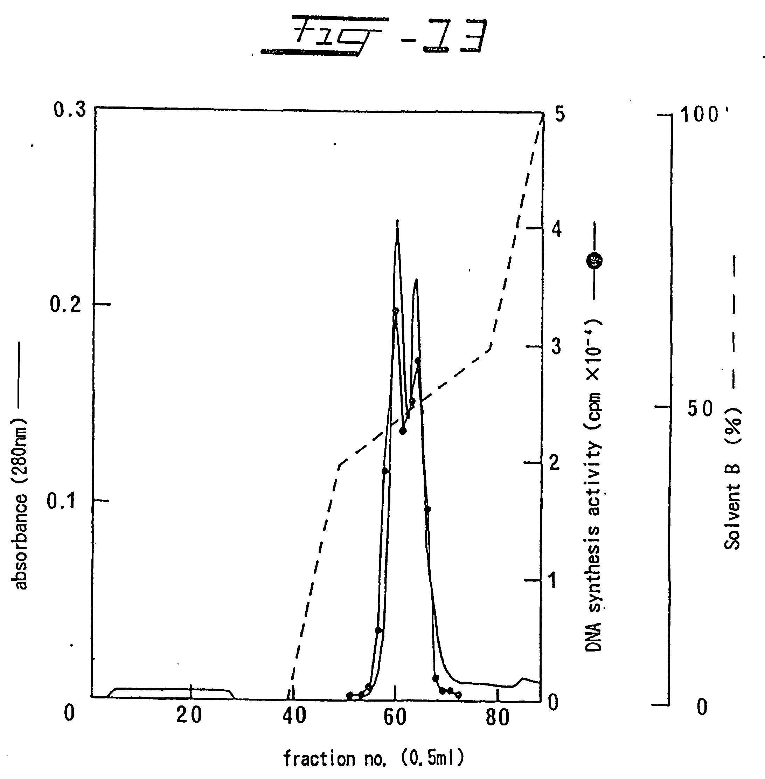

The construction schematics of the human leukocyte-derived HGF expression vector pBPMT[LeHGF] (FERM BP-2897 deposited at the Fermentation Research Institute, Agency of Industrial Science and Technology, Ministry of International Trade and Industry, Japan) and pBPMT[dLeHGF] (FERM BP-2898) for mouse C127 cells are shown in Fig. 5. The plasmids pBS[LeHGF] and pBS[dLeHGF] as obtained in Example 1 were digested with restriction enzymes XbaI and Sall and given blunt ends with T4DNA polymerase, after which they were mixed with expression vector pBPMT for C127 cells digested in advance with restriction enzyme EcoRV, and ligated by T4DNA ligase to give human HGF expression vectors pBPMT[LeHGF] (FERM BP-2897) and pBPMT[dLeHGF] (FERM BP-2898) respectively. The thus-obtained human leukocyte-derived HGF expression vectors have human leukocyte-derived HGF gene between MT-1 promotor and poly(A) additional signal of SV40 early gene, and mouse C127 cells transfected with these expression vectors are transformed by bovine papilloma virus (BPV). Selection of the transformed cells can be done by a neo chimera gene, in which the neo gene of transposon Tn5 (Gene, *19*, 329, 1982) was inserted between the promoter sequence and the poly(A) additional signal of thymidine kinase (HSV1 TK) gene derived from herpes simplex virus type I.

### 2) Transformation of mouse C127 cell and expression of human HGF gene:

The human leukocyte-derived HGF expression vectors pBPMT[LeHGF] (FERM BP-2897) and pBPMT[dLeHGF] (FERM BP-2898) were introduced into mouse C127 cell by the Wigler et al method (Cell, *11*, 223, 1977).

pBPMT[LeHGF] (FERM BP-2897) and pBPMT[dLeHGF] (FERM BP-2898) as obtained in 1) (29 µg) were each dissolved in 240 µl of 0.5 M calcium chloride, and 240 µl of 2 × HEPES buffer, pH 7.1 containing 20 mM HEPES, 280 mM NaCl and 1.5 mM sodium phosphate was added thereto while stirring. Stirring was continued at room temperature for 30 minutes to produce coprecipitation of the plasmid and calcium phosphate. C127 cells (5 × 10⁵) were cultured in advance at 37°C for 24 hours in the presence of 5% CO₂ in DMEM medium (Nissui Pharmaceutical) supplemented with 10% fetal bovine serum (Gibco) and 10 mM glutamine. After replacement of the culture medium, the coprecipitation of the plasmid and calcium phosphate was added thereto and the mixture was left standing at room temperature for 20 minutes. After further incubation at 37°C for 4 hours, the medium was removed and 1 × HEPES buffer added with 15% glycerine was added, and the mixture was left standing at room temperature for 5 minutes. After the cells were washed with the medium, the medium was replaced and further incubation at 37°C for 2 days was conducted. The cells were diluted ten times, and using the same medium containing G418 (Sigma Corp.) at the concentration of 1 mg/ml, cultivation at 37°C for 7 days was conducted in the presence of 5% CO₂ to obtain transformed cells. The cells possessing high HGF activity in the culture supernatant of the obtained cell strains were subjected to screening by the limiting dilution method to obtain human leukocyte-derived HGF high-producing cell strains BPI-14 (pBPMT[LeHGF] (FERM BP-2897)) and BPD-27 (pBPMT[dLeHGF] (FERM BP-2898)). The HGF-producing activity of the cells was 120 thousand unit/ℓ/day and 150 thousand unit/ℓ/day, respectively.

### Example 3

### 1) Construction of human leukocyte-derived HGF expression vector for Chinese hamster CHO cells:

The construction schematics of human leukocyte-derived HGF expression vectors pEVSSV[LeHGF] (FERM BP-2899) and pEVSSV[dLeHGF] (FERM BP-2900) for Chinese hamster CHO cell are shown in Fig. 6. After the plasmids pBS[LeHGF] and pBS[dLeHGF] as obtained in Example 1 were digested with restriction enzymes XbaI and SalI, respectively and given blunt ends by T4DNA polymerase, they were mixed with expression vector pEVSSV for CHO cells, which had been digested with a restriction enzyme EcoRV, and ligated by T4DNA ligase to give human leukocyte-derived HGF expression vectors pEVSSV[LeHGF] (FERM BP-2899) and pEVSSV[dLeHGF] (FERM BP-2900). The thus-obtained human leukocyte-derived HGF expression vectors have human leukocyte-derived HGF gene between the SV40 early promotor and the poly(A) additional signal. Selection of the transformed cells can be done by DHFR chimera gene in which the mouse dihydrofolate reductase (DHFR) gene was ligated with the SV40 early promoter and poly(A) additional signal.

### 2) Transformation of Chinese hamster CHO cell and expression of human leukocyte-derived HGF gene:

The human leukocyte-derived HGF expression vectors pEVSSV[LeHGF] (FERM BP-2899) and pEVSSV[dLeHGF] (FERM BP-2900) were introduced into the CHO DUKX cells deficient in DHFR of Chinese hamster CHO cells in the same manner as in Example 2. The cells having high HGF activity in the culture supernatant of the obtained cell strains were subjected to screening by the limiting dilution method to obtain cells in the culture supernatant, using α-MEM medium (Flow-Laboratory) free of ribonucleoside and deoxyribonucleoside but containing 10% fetal bovine serum (Gibco), 1% glutamine and 50 nM methotrexate. The obtained colony was cultivated in the same culture until the 9th passages to obtain stable human leukocyte-derived HGF high-production strains. The cell strains were grown in the same medium while the concentration of methotrexate was elevated to 100 nM, 250 nM, 500 nM, 750 nM and 1000 nM sequentially, to obtain stable human leukocyte-derived HGF high-production strains EVI-65 (pEVSSV[LeHGF] (FERM BP-2899)) and EVD-104 (pEVSSV[dLeHGF] (FERM BP-2900)). The human leukocyte-derived HGF producing activity of these cells were 90 thousand unit/ℓ /day and 130 thousand unit/ℓ/day, respectively.

### Example 4

### Purification of recombinant human leukocyte-derived HGF from transformed C127 cell culture supernatant:

Recombinant human leukocyte-derived HGF was purified from culture supernatant of human leukocyte-derived HGF-production mouse C127 recombinant cell strain BPD-27 (15 nucleotide deleted HGF production strain) as obtained in Example 2.

### 1) Cation exchange chromatography

Tween 80 was added to a culture solution (500 ml) of BPD-27 strain so that the final concentration became 0.01% and the mixture was filtered through a Sterivex HV filter (Japan Millipore Limited). To the filtrate was added 1/20 volume of 1M Tris-HCl buffer (pH 8.5), which was then poured onto S-Sepharose FF (Pharmacia, column size: inner diameter 1.6 cm, height 5 cm) equilibrated with buffer A (50 mM Tris-HCl, 10 mM HEPES, 2 mM CaCl₂, 150 mM NaCl, 0.01% Tween 80, pH 8.5). After washing adsorbed substance with buffer A, the unadsorbed substance was eluted with the NaCl linear gradient (total amount 100 ml) from 0.15 M to 1.0 M. Fig. 7 shows the chromatogram pattern obtained.

The fractions having HGF activity were collected and used as an S-Sepharose eluate.

### 2) Affinity chromatography

The S-Sepharose eluate was adjusted to pH 7.5 with 1 N acetic acid, diluted with distilled water containing 2-fold volume of 0.01% Tween 80, and equilibrated with buffer B (10 mM Tris-HCl, 0.3 M NaCl, 0.01% Tween 80, pH 7.5), which was then poured onto heparin-Sepharose CL-6B (Pharmacia, column size: inner diameter 1 cm, height 3 cm). After washing the column with buffer B, elution was conducted with the NaCl linear gradient (total amount 30 ml) from 0.3 M to 2.0 M. Fig. shows the chromatogram pattern obtained. The fractions having HGF activity were collected and used as-a heparin eluate.

### 3) Reverse phase HPLC

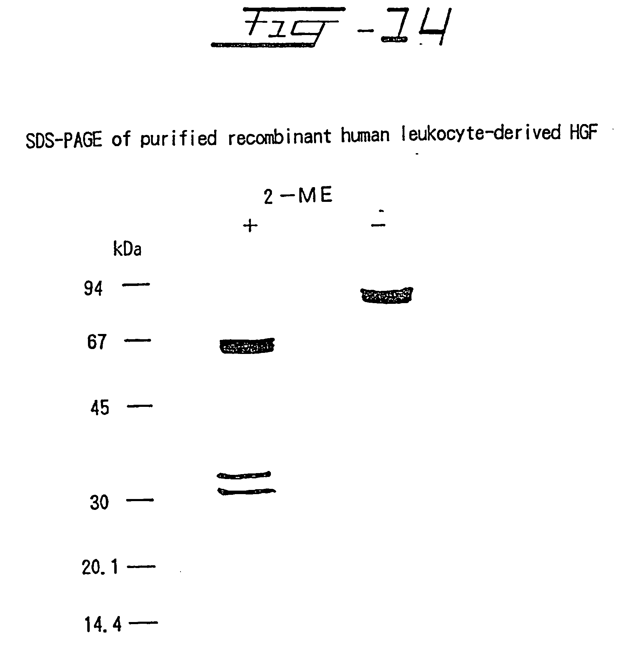

The heparin eluate was poured onto a phenyl 5PW RP column (Toso Corp., inner diameter 0.75 cm, height 7.5 cm) equilibrated with distilled water containing 0.1% TFA (trifluoroacetate, v/v%), and elution was conducted with the 0.1% TFA-containing acetonitrile gradient from 0% to 90%. The recombinant human leukocyte-derived HGF was eluted at about 40% acetonitrile concentration. Fig. 9 shows the chromatogram pattern. Yield of the purified recombinant human HGF was about 20 µg and activity recovery from the culture supernatant was 18%.

### 4) SDS-polyacrylamide gel electrophoresis

The 15 nucleotide deleted type recombinant human leukocyte-derived HGF which was purified by 3 steps of the above-mentioned chromatography was subjected to SDS-polyacrylamide gel electrophoresis under 2-mercaptoethanol reducing and nonreducting conditions. The results are shown in Fig. 10. The purified recombinant HGF showed a single band with 70,000 to 90,000 molecular weight under nonreducing conditions (2-ME(-)), and α-chain with 60,000 to 75,000 molecular weight and β-chain with 30,000 to 40,000 molecular weight under reducing conditions (2-ME(+)). That is, the recombinant HGF is a heterodimer comprising α- and β-chains.

### 5) Hepatocyte growth activity of the recombinant human leukocyte-derived HGF (15 nucleotide deleted type)

Among presently-known *in vitro* assay system, rat primary cultured hepatocytes have liver functions most similar to those *in vivo*. Addition of the purified 15 nucleotide deleted type recombinant human leukocyte-derived HGF to rat hepatocytes as obtained in accordance with the method described-under "Determination of HGF activity" induced strong cell proliferation at a concentration of 1-20 ng/ml. As other factors showing growth activity to this culture strain, there have been known insulin and EGF. The recombinant HGF by itself shows more potent activity than the two and additive action was observed in the presence of these three.

### Example 5

### Transformation and expression of Chinese hamster CHO cell by human leukocyte-derived HGF gene:

Human leukocyte-derived HGF expression vector pEVSSV[dLeHGF] (FERM BP-2900) was introduced into DHFR deficient cells of Chinese hamster CHO cells in accordance with the Wigler et al method (Cell, *11* , 233, 1977). pEVSSV[dLeHGF] plasmid (about 30 µg) was dissolved in 0.5 M calcium chloride (240 µl), and 2 × HEPES buffer (240 µl, pH 7.1) containing 20 mM HEPES, 280 mM sodium chloride and 1.5 mM sodium phosphate, was added thereto while stirring. Stirring was continued at room temperature for 30 minutes to form coprecipitation of plasmid and calcium phosphate. CHO cells (5 × 10⁵) were cultured in α-MEM culture medium (Flow-Laboratory Corp.) containing 10% fetal bovine serum (Gibco) and 1% glutamine in the presence of 5% CO₂ at 37°C for 24 hours. After-medium exchange, coprecipitation of plasmid and calcium phosphate was added thereto, followed by leaving at room temperature for 20 minutes. Incubation was further continued at 37°C for 4 hours, and the medium was removed, 1 × HEPES buffer supplemented with 15% glycerine was added thereto, and the mixture was left standing at room temperature for 5 minutes. Cells were washed with the medium, and the medium was changed, followed by 7 days' cultivation at 37°C to give transformed cells. The obtained cell strains were repeatedly cultivated using α-MEM culture medium (Flow-Laboratory Corp.) containing 10% fetal bovine serum (Gibco) and 1% glutamine but not containing ribonucleoside and deoxyribonucleoside while elevating the concentration of methotrexate to 100 nM, 250 nM, 500 nM, 750 nM, 1 µM and 2 µM sequentially, to obtain stable HGF high-production strains. Cloning selection was conducted for the obtained human leukocyte-derived HGF high-production recombinant cells to obtain stable human leukocyte-derived HGF-production cell strain 515C. The HGF producing activity of the cells was about 800 thousand unit/ℓ/day.

### Example 6

### Purification of recombinant human leukocyte-derived HGF from transformed CHO cell culture supernatant:

Human leukocyte-derived HGF production Chinese hamster CHO recombinant cell strain 515C (15 nucleotide deleted type HGF production strain) as obtained in Example 5 was cultured in α-MEM culture medium (Flow-Laboratory_Corp.) free of ribonucleoside and deoxyribonucleoside but containing 10% fetal bovine serum (Gibco), 1% glutamine and 2 µM methotrexate, from the culture supernatant of which recombinant human leukocyte-derived HGF was purified.

### 1) Cation exchange chromatography

Tween 80 was added to the culture (500 ml) of 515C strain so that the final concentration became 0.01%, and the mixture was filtered through a Sterivex HV filter (Japan Millipore Limited). To the filtrate was added 1/20 volume of 1M Tris-HCl buffer (pH 8.5), after which it was poured onto S-Sepharose FF (Pharmacia, column size: inner diameter 1.6 cm, height 5 cm) equilibrated with buffer C (50 mM Tris-HCl, 0.01% Tween 80, pH 8.5) containing 150 mM NaCl. After washing the buffer C column with buffer C containing 150 mM NaCl and buffer C containing 400 mM NaCl (marked by arrow A in Fig. 11), it was eluted with buffer A containing 1 M NaCl (marked by arrow B in Fig. 11). Fig. 11 shows the chromatogram pattern obtained. The peak portions eluted with buffer C containing 1 M NaCl (marked by <―> in Fig. 11) were collected and used as an S-Sepharose eluate.

### 2) Affinity chromatography

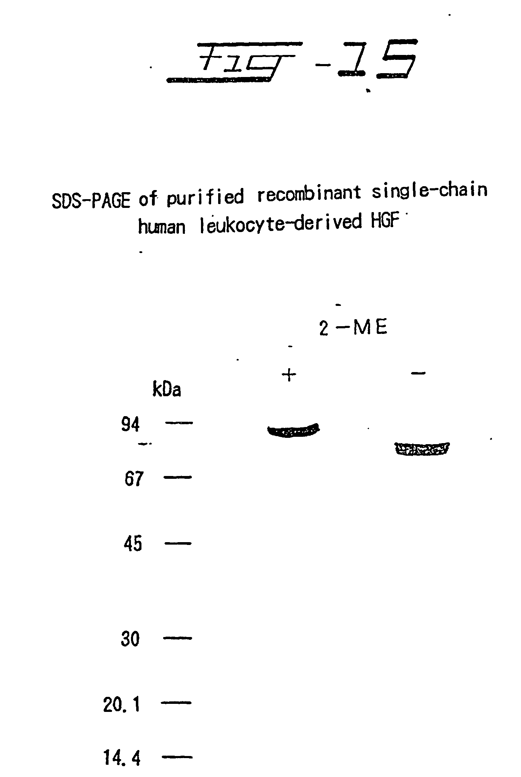

The S-Sepharose eluate was adjusted to pH 7.5 with 1 N hydrochloric acid, diluted with distilled water containing 2-fold volume of 0.01% Tween 80, and then poured onto heparin-Sepharose CL-6B (Pharmacia, column size: inner diameter 1 cm, height 5 cm) equilibrated with buffer B (10 mM Tris-HCl, 0.3 M NaCl, 0.01% Tween 80, pH 7.5). After washing the column with buffer B, elution was conducted with the NaCl linear gradient (total amount 40 ml) from 0.3 M to 2.0 M. Fig. 12 shows the chromatogram pattern obtained. The fractions having HGF activity were collected and used as a heparin eluate.

### 3) Hydrophobic chromatography

The heparin eluate was poured onto a phenyl 5PW column (Toso Corp., inner diameter 0.75 cm, height 7.5 cm) equilibrated with 20 mM phosphate buffer, pH 7.0, containing 4M NaCl, and elution was conducted with the gradient from solvent A: 20 mM phosphate buffer containing 4M sodium chloride (pH 7.0) to solvent B: 20 mM phosphate buffer containing 50% ethylene glycol (pH 7.0). HGF activity was eluted at about 2M NaCl, 25% ethylene glycol. Fig. 13 shows its chromatogram. Yield of the purified recombinant human leukocyte-derived HGF was about 500 µg and activity recovery from the culture supernatant was 25%. 4) Properties of the purified recombinant human leukocyte-derived HGF

Biological, chemical and physicochemical properties of the recombinant human leukocyte-derived HGF as obtained in 3) above were measured.

### ① SDS-polyacrylamide gel electrophoresis

The recombinant HGF was subjected to SDS-polyacrylamide gel electrophoresis under 2-mercaptoethanol reducing and nonreducing conditions. After the electrophoresis, gel was stained by the silver staining method. The results are summarized in Fig. 14. The recombinant HGF showed a single band with 70,000 to 90,000 molecular weight under nonreducing conditions and α-chain with 60,000 to 75,000 molecular weight and β-chain with 30,000 to 40,000 molecular weight under reducing conditions. The β-chain further showed two bands which indicates difference of the number of the bound sugar chains.

### ② Sugar composition analysis (neutral sugar and amino sugar)

After evaporation to solid of the purified recombinant HGF, it was subjected to hydrolysis in the presence of 2.5 N trifluoroacetate at 110°C for 6 hours. The hydrolyzate was evaporated to dryness and redissolved in water to give a sample. The sample was subjected to sugar composition analysis by HPLC using an anion-exchange resin. As a result, fucose, galactose, mannose and N-acetyl glucosamine were detected, which confirmed that the recombinant HGF was a glycoprotein.

### ③ Biological activity

Hepatocyte growth activity of the purified recombinant HGF was determined in accordance with the method described under "Determination of HGF activity". As a result, relative activity of the purified recombinant HGF was found to be 200-500 thousand unit/mg.

### Example 7

### Production of recombinant single-chain human leukocyte-derived HGF (15 nucleotide deleted type HGF)

The recombinant human leukocyte-derived HGF production CHO 515C (15 nucleotide deleted type HGF) strain as obtained in Example 5 was cultured in an α-MEM culture medium (Flow-Laboratory Corp.) free of ribonucleoside and deoxyribonucleoside but containing 10% fetal bovine serum (Gibco), 1% glutamine and 2 µM methotrexate at 37°C in 5% CO₂ until the cells became confluent. After cultivation, the culture solution was removed and the cells were washed twice with PBS. Thereto was added α-MEM culture medium free of ribonucleoside and deoxyribonucleoside but supplemented with 1% glutamine, 500 µM methotrexate and 400 unit/ml aprotinin which is a protease inhibitor, and the cells were cultured at 37°C in 5% CO₂. After cultivation for about 1 day, the culture supernatant was harvested, and recombinant HGF was purified by chromatography procedure in the like manner as in Example 6. Activity recovery from the culture supernatant was about 15%.

The purified 15 nucleotide deleted type recombinant human leukocyte-derived HGF was subjected to SDS-acrylamide gel electrophoresis. The results are summarized in Fig. 15. The purified recombinant HGF showed a single band with 70,000 to 90,000 molecular weight under nonreducing conditions and a single band with 80,000 to 95,000 molecular weight under 2-mercaptoethanol reducing conditions, which indicated that the recombinant HGF obtained was of single chain. Furthermore, biological activity of said recombinant single-chain HGF was measured, which comprised measurement of growth activity to primary culture of rat hepatocytes as described under "Determination of HGF activity". As a result, the recombinant single-chain HGF showed hepatocyte growth activity and relative activity thereof was almost the same as one obtained in Example 6, 4), which was 200-500 thousand unit/mg.

## Claims

1. A method of producing a hepatocyte growth factor (HGF) which comprises;
1) transforming mammalian cells with a recombinant expression vector which has a promoter, a gene coding for an amino acid sequence shown in Fig. 2 and the DHFR gene,
2) culturing the transformed cells in the presence of successively elevated concentrations of methotrexate, and
3) harvesting the HGF from conditioned media of the cells obtained in 2).

2. The method according to claim 1, wherein the mammalian cells are CHO cells, and the promoter is the SV40 early promoter.

3. A HGF high-production transformant strain obtainable by the procedure which comprises:
1) transforming mammalian cells with a recombinant expression vector which has a promoter, a gene coding for an amino acid sequence shown in Fig. 2 and the DHFR gene,
2) culturing the transformed cells in the presence of successively elevated concentrations of methotrexate, and
3) establishing a HGF high-production strain.

4. The HGF high-production transformant strain according to claim 3, wherein the mammalian cells are CHO cells.

5. A method of producing HGF by harvesting the HGF from the conditioned media of the strain according to claim 3 or 4.

## Patentansprüche

1. Verfahren zur Herstellung eines Hepatocytenwachstumsfaktors (HGF), umfassend:
1) Transformieren von Säugerzellen mit einem rekombinanten Expressionsvektor, der einen Promotor, ein Gen, das für eine in Figur 2 gezeigte Aminosäuresequenz codiert, und das DHFR-Gen enthält;
2) Kultivieren der transformierten Zellen in Gegenwart von sukzessive erhöhten Konzentrationen an Methotrexat; und
3) Ernten des HGF aus konditionierten Medien der in 2) erhaltenen Zellen.

2. Verfahren gemäß Anspruch 1, wobei die Säugerzellen CHO-Zellen sind und der Promotor der frühe SV40-Promotor ist.

3. Transformanter Stamm mit hoher HGF-Produktion, erhältlich durch das Verfahren, das Folgendes umfasst:
1) Transformieren von Säugerzellen mit einem rekombinanten Expressionsvektor, der einen Promotor, ein Gen, das für eine in Figur 2 gezeigte Aminosäuresequenz codiert, und das DHFR-Gen enthält;
2) Kultivieren der transformierten Zellen in Gegenwart von sukzessive erhöhten Konzentrationen an Methotrexat; und
3) Etablieren eines Stammes mit hoher HGF-Produktion.

4. Transformanter Stamm mit hoher HGF-Produktion gemäß Anspruch 3, wobei die Säugerzellen CHO-Zellen sind.

5. Verfahren zur Herstellung von HGF durch Ernten des HGF aus dem konditionierten Medium des Stammes gemäß Anspruch 3 oder 4.

## Revendications

1. Procédé pour la production d'un facteur de croissance hépatocyte (FCH, en Anglais, hepatocyte growth factor, HGF) qui comprend :
1) transformer des cellules mammifères avec un vecteur d'expression recombinant qui possède un promoteur, un gène codant pour une séquence d'acides aminés comme cela apparaît sur la Figure 2 et le gène DHFR,
2) mettre en culture les cellules transformées en présence de concentrations successivement élevées de méthotrexate, et
3) recueillir le FCH à partir de milieux conditionnés des cellules obtenues en 2).

2. Procédé selon la revendication 1, dans lequel les cellules mammifères sont des cellules CHO, et le promoteur est le promoteur précoce de SV40.

3. Souche de transformant haute production en FCH pouvant être obtenue par la procédure qui comprend :
1) transformer des cellules mammifères avec un vecteur d'expression recombinant qui possède un promoteur, un gène codant pour une séquence d'acides aminés comme cela apparaît sur la Figure 2 et le gène DHFR ,
2) mettre en culture les cellules transformées en présence de concentrations successivement élevées de méthotrexate, et
3) établir une souche haute production en FCH.

4. Souche de transformant haute production en FCH selon la revendication 3, dans laquelle les cellules mammifères sont des cellules CHO.

5. Procédé pour la production FCH par recueil de FCH à partir des milieux conditionnés de la souche selon la revendication 3 ou 4.
